# FASCICULE DE BREVET EUROPEEN

(11) **EP 3 283 162 B1**
(45) Date de publication et mention de la délivrance du brevet: **24.02.2021**
(21) Numéro de dépôt: 16717351.7
(22) Date de dépôt: 15.04.2016
(51) Int. Cl.: A61M 39/22, F16K 11/08, A61M 39/10

(54) **ROBINET MEDICAL, KIT COMPRENANT UN TEL ROBINET ET METHODE DE PREPARATION D'UN MELANGE OU D'UNE EMULSION.**
MEDIZINISCHES VENTIL, KIT MIT SOLCH EINEM VENTIL UND VERFAHREN ZUR HERSTELLUNG EINES GEMISCHES ODER EINER EMULSION
MEDICAL VALVE, KIT COMPRISING SUCH A VALVE, AND METHOD FOR PREPARING A MIXTURE OR AN EMULSION

(30) Priorité: 15.04.2015 FR 1553325
(43) Date de publication de la demande: 21.02.2018
(73) Titulaire: Guerbet, 93420 Villepinte (FR)
(72) Inventeur: ALLARD, Ludovic, 69390 Millery (FR)
(74) Mandataire: Lavoix
(86) Numéro de dépôt international: PCT/EP2016/058453
(87) Numéro de publication internationale: WO 2016/166346

(56) Documents cités:
- WO-A1-2008/009946
- WO-A1-2012/024370
- WO-A1-2014/090958
- US-A- 2 703 586
- US-A- 4 217 933
- US-A- 4 807 666
- US-A1- 2013 030 348
- US-A1- 2014 276 215

## Description

L'invention concerne un robinet médical, un kit de préparation d'un produit à injecter, préférentiellement un mélange ou une émulsion, comprenant un tel robinet médical, et une méthode de préparation d'un mélange ou d'une émulsion destinée à l'injection chez un patient mise en œuvre à l'aide d'un tel kit de préparation.

Depuis près de trente ans, l'huile iodée Lipiodol® est utilisée en radiologie interventionnelle. Ce produit se caractérise par sa propension à être capté sélectivement par les tumeurs hépatiques. Cette huile iodée est donc utilisée comme vecteur d'agent anticancéreux pour le traitement du carcinome hépatocellulaire dans une technique appelée « chimio-embolisation intra-artérielle » ou « TransArterial ChemoEmbolisation » (c-TACE) en anglais (Nakamura et al.: Radiology, 1989 ; 170:783-6 et J.M. Idée - B. Guiu : Critical Reviews in Oncology/Hematology, 2013 ; 88(3):530-49). Les huiles iodées et en particulier Lipiodol® sont aussi connues pour induire une embolisation transitoire de la circulation artérielle provoquant ainsi un ralentissement de celle-ci. Etant donné que la plupart des agents anticancéreux sont solubles dans l'eau, la forme « émulsion », qui est adaptée pour le mélange de deux phases non solubles l'une dans l'autre, apparait comme étant la plus judicieuse pour mélanger une huile iodée et un agent anticancéreux. Elle semble être la plus adaptée pour transporter et délivrer dans une tumeur un agent anticancéreux, trop toxique et pas assez efficace quand il est administré non émulsifié par voie intra-artérielle ou par voie systémique.

Pour réaliser une c-TACE, un radiologue interventionnel prépare extemporanément l'émulsion, juste avant l'injection. Il utilise classiquement deux seringues de 50 ml connectées à un robinet 3 ports pourvu de deux connecteurs femelles auxquels sont connectées les seringues et d'un connecteur mâle auquel un cathéter ou un micro-cathéter peut être fixé. L'une des seringues contient une solution d'un agent anticancéreux tandis que l'autre seringue contient une huile iodée comme le Lipiodol®. L'émulsion est obtenue par exemple après dix passages successifs rapides du contenu d'une seringue à l'autre, à l'aide d'un robinet trois voies. L'émulsion est alors transférée dans l'une des deux seringues de mélange, la seringue de mélange vide est déconnectée du robinet et une seringue d'injection est alors mise à la place de la seringue de mélange qui a été enlevée. Une petite quantité d'émulsion est transférée dans la seringue d'injection en actionnant le piston de la seringue de mélange restante. L'injection est réalisée en dix minutes dans la branche droite ou gauche de l'artère hépatique d'un patient irriguant la plus grande partie de la tumeur.

Etant donné qu'il y a parfois plusieurs injections successives qui sont réalisées, l'émulsion restante dans la seringue de mélange est parfois mélangée à nouveau comme décrit ci-dessus, après déconnexion de la seringue d'injection et reconnexion de la seconde seringue de mélange. Une nouvelle injection est réalisée après le transfert de l'émulsion dans l'une des deux seringues de mélange, la déconnexion de la seringue de mélange vide du robinet, la connexion d'une seringue d'injection à la place de la seringue de mélange qui a été enlevée et le transfert d'une petite quantité d'émulsion dans la seringue d'injection en actionnant le piston de la seringue de mélange restante.

Les robinets en matériau plastique actuellement sur le marché qui sont destinés à un usage médical et en particulier qui sont destinés à réaliser la préparation d'une émulsion qui sera injectée à un patient dans le cadre d'une chimio-embolisation intraartérielle, sont majoritairement des robinets trois ports et présentent différents problèmes gênants pour le radiologue interventionnel pratiquant cette technique.

Les robinets en matériau plastique destinés à un usage médical, tels que connus de FR 2 804 609, ne supportent le plus souvent pas quand la manipulation des dispositifs qui leur sont connectés génère une tension importante sur les ports de connexion. Ces robinets cassent et ainsi des fuites apparaissent au niveau de la base de ces ports de connexion. Cette fragilité peut aussi être accrue par les attaques chimiques de certains produits passant dans ces robinets.

Ces robinets en matériau plastique sont fabriqués par injection d'un matériau plastique dans un moule. Le renforcement de ces robinets pour améliorer leur solidité a comme inconvénient d'augmenter le risque qu'au moment de leur fabrication des retassures se forment dans leur corps. Les retassures sont des défauts de fabrication, et plus précisément d'injection, qui apparaissent lors du refroidissement de la pièce plastique, sous l'effet du retrait de la matière plastique. Elles se présentent sous l'aspect d'une dépression située près des parties massives de la pièce plastique et sur les surfaces planes. D'une injection à l'autre, la position des retassures ne varie pas puisqu'elle est liée au design de la pièce et non aux paramètres d'injection. Ces retassures augmentent le risque de survenue de fuites dans ces dispositifs.

WO 2008/057946 décrit un robinet médical composé d'un corps comprenant un port d'entrée et deux ports de sortie, et d'un boisseau qui s'insère dans le corps et permet la communication entre le port d'entrée et les ports de sortie du robinet par rotation. Un des problèmes techniques qui se pose lors de la fabrication de ce robinet, est que lors de l'étape de stérilisation à la vapeur, la chaleur a tendance à déformer et à rétrécir le corps du robinet, qui est formé de polychlorure de vinyle (PVC). Ce problème peut être résolu en utilisant alors un matériau plus rigide, tel que du polycarbonate ou de l'acier inoxydable. Ces matériaux peuvent toutefois conduire à d'autres problèmes, tels qu'une étanchéité insuffisante des ports à l'entrée et sortie des fluides. Il n'est pas non plus fait mention dans ce document des problèmes liés aux retassures.

Il est également connu de l'état de la technique, notamment du brevet EP 1 504 207, des robinets de perfusion à haute pression. Ce brevet décrit un robinet à haute pression constitué d'un corps de robinet et d'un boisseau muni d'une ailette. Le corps du robinet comprend un port d'entrée, au moins un port de sortie et une collerette solidaire des ports d'entrée et de sortie du robinet et du fût central de ce robinet, permettant de rigidifier le corps du robinet afin de mieux résister aux contraintes mécaniques et chimiques que subit le robinet. Cependant, un tel robinet présente toujours comme inconvénient de former des retassures à l'intérieur du logement du boisseau, ou fût central, lors de sa fabrication et donc des problèmes de fuites potentiels.

Il est également connu de l'état de la technique, notamment du brevet US 4,807,666, de renforcer la structure d'un robinet par la présence d'un voile entre les bords plus épais de la collerette et le fût du robinet. Un tel voile aura pour effet, lors du refroidissement et de la prise de retrait du matériau plastique, « d'aspirer » la matière plastique de la zone la plus chaude vers la zone la plus froide. Ce phénomène, bien décrit dans l'état de la technique, provoque une retassure Qui apparait dans le fût du corps de robinet, qui est globalement une zone plus difficile à refroidir que l'extérieur du fût.

C'est à ces inconvénients qu'entend remédier l'invention en proposant un nouveau robinet médical dont la qualité de fabrication est améliorée.

A cet effet, l'invention concerne un robinet médical comprenant un corps muni d'au moins deux connecteurs femelles et d'un connecteur mâle, un boisseau mobile monté dans le corps, muni d'un levier de rotation et comprenant un canal de circulation de fluide, et une collerette de renfort solidaire d'au moins deux des connecteurs, la collerette de renfort étant espacée du corps pour former une zone d'ajourage autour du corps central.

Grâce à l'invention, les contraintes mécaniques qui apparaissent lors de l'utilisation du robinet médical sont concentrées aux intersections de la collerette avec les connecteurs, dans une zone éloignée du corps central du robinet.

De manière préférentielle, selon l'invention, le corps du robinet comprend :
- au moins deux connecteurs femelles et un connecteur mâle, préférentiellement trois connecteurs femelles et un connecteur mâle,
- un fût dans lequel le boisseau vient s'insérer et
- la collerette de renfort.

Selon des aspects avantageux mais non obligatoires de l'invention, un tel robinet peut incorporer une ou plusieurs des caractéristiques suivantes, prises dans toute combinaison techniquement admissible:
- Le robinet comprend quatre connecteurs dont trois connecteurs femelles, et la collerette de renfort est solidaire de trois ou quatre connecteurs.
- Le robinet médical comprend au moins deux moyens de préhension adaptés au positionnement d'un doigt, et les moyens de préhension sont situés sur la collerette de renfort.
- Au moins un des connecteurs femelles est adapté pour recevoir une seringue d'injection et comprend un détrompeur permettant d'empêcher le montage sur ce connecteur d'une seringue de mélange, et ce détrompeur est formé de deux cornes s'étendant à partir de la collerette de renfort et prévues de part et d'autre du connecteur femelle adapté pour recevoir une seringue d'injection et formant par rapport à ce connecteur des espaces d'insertion.
- Les cornes permettent d'empêcher le montage de seringues pourvues d'un moyen rendant incompatible leur connexion sur le connecteur femelle adapté pour recevoir une seringue d'injection.
- La collerette de renfort est réalisée dans le même matériau que le corps.
- La collerette de renfort est réalisée dans un matériau différent de celui du corps et de celui du boisseau.
- La collerette de renfort est réalisée en un matériau chargé en fibres et surmoulée sur le corps.
- Le boisseau est réalisé dans un matériau plus tendre que le corps et/ou que la collerette de renfort.
- La collerette de renfort a une largeur supérieure à son épaisseur, de préférence une largeur 3 à 10 fois plus importante que son épaisseur.

On considère que l'invention concerne également un ensemble médical comprenant un robinet tel que mentionné ci-dessus, au moins une seringue de mélange et au moins une seringue d'injection.

L'invention concerne également un kit de préparation d'un produit à injecter, préférentiellement un mélange ou une émulsion, comprenant un robinet médical tel que mentionné ci-dessus, deux seringues de mélange adaptées pour être connectées à un premier et à un deuxième connecteur femelles du robinet, et une seringue d'injection adaptée pour recueillir au moins une partie du produit obtenu par le mélange des contenus des seringues de mélange réalisé par un mouvement de va-et-vient des pistons desdites seringues de mélange et adaptée pour être connectée à l'un des connecteurs femelles du robinet.

Selon des aspects avantageux mais non obligatoires de l'invention, un tel robinet peut incorporer une ou plusieurs des caractéristiques suivantes, prises dans toute combinaison techniquement admissible:
- L'un des connecteurs femelles du robinet comprend un détrompeur et en ce que les seringues de mélange comprennent un moyen rendant incompatible leur connexion avec ledit connecteur femelle.
- Les seringues de mélange comprennent des ailettes à leur extrémité.

L'invention concerne également une méthode de préparation d'un mélange ou d'une émulsion destiné à l'injection chez un patient, comprenant des étapes consistant à :
a) connecter deux seringues de mélange à deux connecteurs femelles correspondants d'un robinet médical tel que mentionné ci-dessus ;
b) mélanger une solution aqueuse contenue dans l'une des seringues de mélange avec une huile contenue dans l'autre seringue de mélange après avoir positionné un boisseau du robinet médical de manière à mettre en communication fluidique les seringues de mélanges l'une avec l'autre et en effectuant un mouvement de va-et-vient des pistons de ces seringues de mélange jusqu'à l'obtention d'un mélange ou d'une émulsion ;
c) connecter une seringue d'injection à un connecteur spécifique du robinet médical ou à la place des seringues de mélange;
d) transférer une partie ou la totalité du mélange ou de l'émulsion obtenu à l'étape b) dans la seringue d'injection, après avoir positionné le boisseau de manière à mettre en communication l'une des seringues de mélange avec la seringue d'injection.

Selon un aspect avantageux mais non obligatoire de l'invention, une telle méthode de préparation peut comprendre en outre une étape supplémentaire consistant à connecter un dispositif aval, de préférence un cathéter ou un micro-cathéter, à un connecteur mâle du robinet médical, cette étape étant soit concomitante à l'étape a), soit postérieure à l'étape b), soit concomitante à l'étape c), soit postérieure à l'étape d).

L'invention sera mieux comprise et d'autres avantages de celle-ci apparaitront plus clairement à la lumière de la description qui va suivre d'un robinet médical, d'un kit de préparation d'un produit à injecter, préférentiellement d'un mélange ou d'une émulsion, et d'une méthode de préparation d'un produit, préférentiellement un mélange ou une émulsion conformes à son principe, faite à titre d'exemple non limitatif en référence aux dessins annexés dans lesquels :
- la figure 1 est une vue en perspective d'un robinet médical conforme à l'invention ;
- la figure 2 est une vue de dessous du robinet médical de la figure 1 ;
- la figure 3 est une coupe du robinet médical des figures 1 et 2, selon un plan comprenant les axes centraux de quatre connecteurs du robinet médical;
- la figure 4 est une vue en perspective d'un boisseau du robinet médical des figures 1 à 3 ;
- la figure 5 est une vue en perspective, selon un autre angle, du boisseau de la figure 4 ;
- la figure 6 est une vue en perspective, selon un autre angle, d'un autre mode de réalisation d'un boisseau;
- la figure 7 est une vue en perspective du robinet médical de la figure 1, dont le boisseau est omis;
- la figure 8 est une coupe du robinet médical des figures 1 et 2, selon un plan passant par la base d'un levier du boisseau;
- la figure 9 est une vue en perspective d'un robinet médical conforme à un second mode de réalisation de l'invention.
- la figure 10 est une vue en perspective du robinet médical des figures 1 à 3, d'un cathéter connecté sur un connecteur du robinet médical et une seringue de mélange positionnée devant un connecteur femelle du robinet médical;
- la figure 11 est une vue en perspective de l'extrémité d'une seringue de mélange;
- la figure 12 est une vue en perspective d'une partie d'un kit de préparation conforme à l'invention.

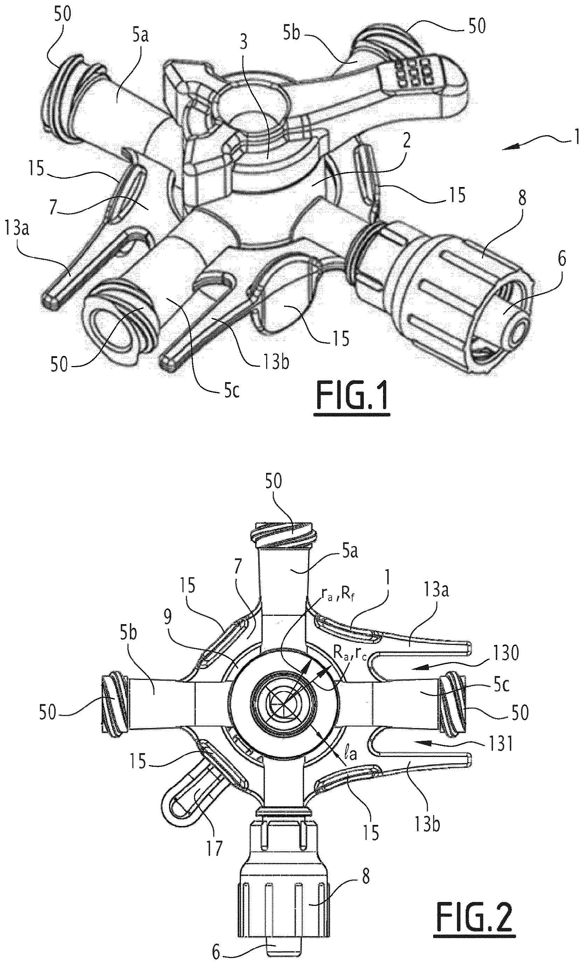

Les figures 1 à 3 représentent un robinet médical 1 permettant de préparer un mélange, préférentiellement une émulsion, destiné à être injecté à un patient. Le robinet médical 1 comprend un corps 2 creux à partir duquel s'étendent trois connecteurs femelles 5a, 5b et 5c et un connecteur mâle 6, préférentiellement un connecteur luer. Le corps 2 comprend un fut de forme cylindrique délimitant un alésage central 200 à partir duquel s'étendent quatre conduits 2a, 2b, 2c et 2d passant à travers le fut et parcourant respectivement les connecteurs 5a, 5b, 5c et 6.

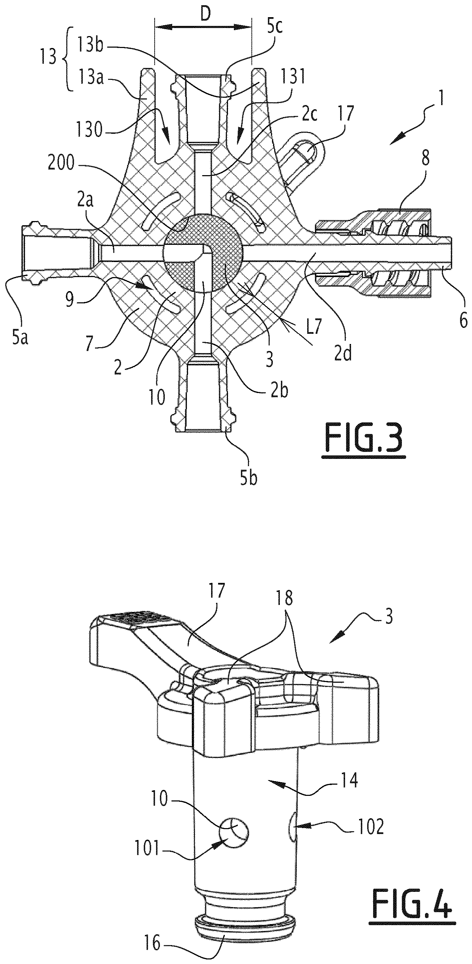

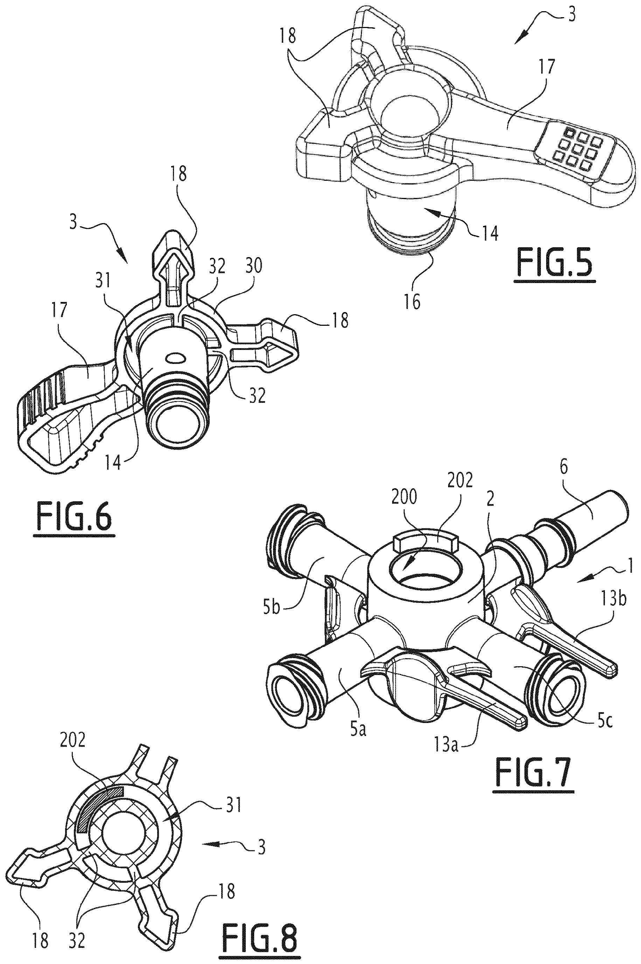

Le robinet 1 comprend un boisseau 3 représenté notamment sur les figures 4 et 5, monté rotatif dans l'alésage 200. Le boisseau 3 comprend un canal de circulation de fluide 10 permettant de mettre en communication les conduits 2a, 2b, 2c et 2d selon un mode décrit ci-après. Le boisseau 3 comprend une paroi cylindrique externe 14 permettant de réaliser l'étanchéité du robinet 1 avec l'alésage 200. Une extrémité axiale du boisseau 3 comprend une collerette 16 permettant son maintien dans le corps 2 par clippage.

Il existe d'autres solutions de maintien du boisseau 3 dans le corps 2, telles que le bouterollage ou l'ajout d'une pièce de maintien. Par « bouterollage », on entend, au sens de la présente invention, la création d'une liaison mécanique entre le boisseau 3 et le corps 2 entrainant la déformation partielle du boisseau 3.

Le boisseau 3 comprend de préférence un levier 17 actionnable par un utilisateur permettant la rotation du boisseau 3 autour de son axe central.

Selon un aspect optionnel de l'invention, l'un des connecteurs femelles, le connecteur 5c dans l'exemple représenté, comprend un détrompeur 13. Par «détrompeur», on entend désigner un dispositif mécanique permettant d'éviter les erreurs de branchement en fournissant une indication visuelle et en définissant une configuration mécanique qui empêche l'utilisation d'éléments non désirés. Dans l'exemple représenté, le détrompeur 13 comprend deux cornes 13a et 13b prévues de part et d'autre du connecteur femelle 5c et qui définissent deux espaces d'insertion 130 et 131 situés respectivement entre les cornes 13a et 13b et le connecteur 5c.

Le détrompeur 13 permet préférentiellement d'empêcher la connexion d'une seringue dont le volume est supérieur à une valeur prédéterminée, grâce à la géométrie des cornes 13a et 13b et la largeur des espaces 130 et 131 ou d'une seringue pourvue d'un moyen rendant incompatible sa connexion avec le connecteur comprenant ce détrompeur 13 et/ou ces cornes 13a et 13b du fait de l'incompatibilité entre ce moyen et la configuration mécanique définie par le détrompeur 13 et/ou les cornes 13a et 13b de ce détrompeur 13. Encore plus préférentiellement, le détrompeur 13 et de manière avantageuse, les cornes 13a et 13b de ce détrompeur 13, permettent d'empêcher le montage de seringues 20 et 21, préférentiellement des seringues de mélange, pourvues d'un moyen rendant incompatible leur connexion avec le connecteur comprenant ce détrompeur 13 et/ou les cornes 13a et 13b de ce détrompeur. Préférentiellement, quand les cornes 13a et 13b empêchent la connexion d'une seringue pourvue d'un moyen rendant incompatible sa connexion avec le connecteur femelle 5c comprenant ce détrompeur 13 et/ou ces cornes 13a et 13b du fait de l'incompatibilité entre ce moyen et la configuration mécanique définie par le détrompeur et/ou les cornes de ce détrompeur, elles ne dépassent pas de plus de 5 mm le bout du connecteur femelle 5c. De préférence, quand les cornes 13a et 13b empêchent la connexion d'une seringue dont le réservoir a un diamètre supérieur à une valeur seuil correspondant à un volume maximal, préférentiellement d'une seringue de mélange, elles dépassent de plus de 5 mm le bout du connecteur femelle.

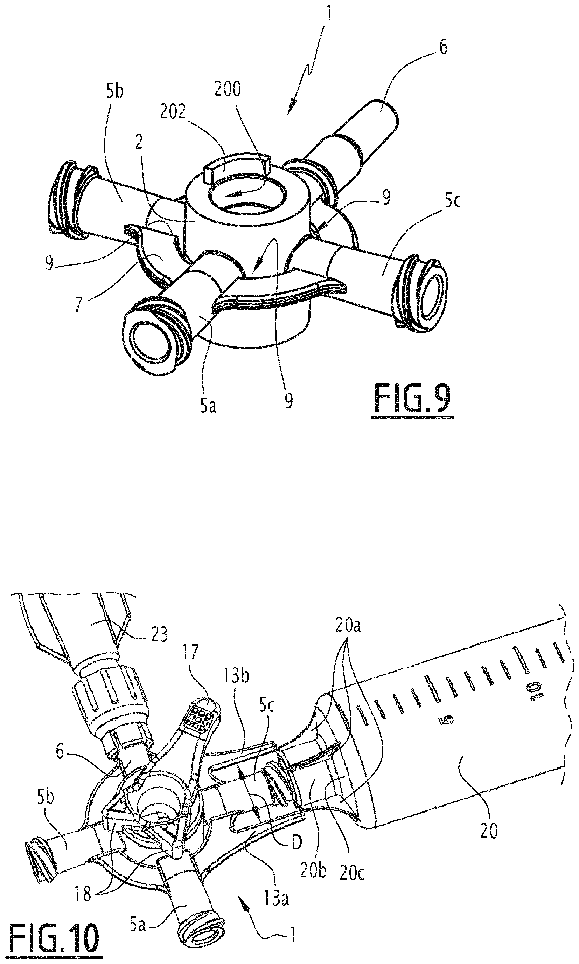

Dans l'exemple représenté à la figure 10, la seringue de mélange 20 est pourvue de moyens rendant incompatible sa connexion avec le connecteur 5c comprenant le détrompeur 13. Dans ce mode de réalisation, la seringue de mélange 20 comprend à son extrémité des ailettes 20a, formant les moyens rendant incompatible la connexion de la seringue de mélange 20 avec le connecteur 5c comprenant un détrompeur. Pour effectuer le vissage d'une seringue de mélange sur un connecteur femelle, préférentiellement du type luer, il faut réaliser entre un et deux tours en fonction du connecteur. Ainsi, la seringue de mélange 20 comprend au moins une ailette 20a, préférentiellement au moins deux ailettes 20a. Cela permet que le détrompeur 13 assure sa fonction. L'extrémité de la seringue de mélange 20 est formée par un connecteur luer comprenant un filetage mâle interne prévu sur une jupe périphérique 20b. Les ailettes 20a sont prévues sur la surface externe de la jupe 20b. Comme montré sur la figure 9, les ailettes 20a créent un encombrement à l'extrémité de la seringue de mélange 20, autour de la jupe 20b du filet mâle du connecteur luer, et qui est supérieur à la distance D entre les cornes 13a et 13b. De manière plus préférentielle, la seringue de mélange 20 comprend à son extrémité quatre ailettes 20a positionnées à 90° les unes par rapport aux autres. Cela permet d'assurer un meilleur équilibre dimensionnel du corps de la seringue ainsi qu'une meilleure ergonomie en termes de préhension de celle-ci. De manière préférentielle, les ailettes 20a de la seringue de mélange 20 ont une forme qui permet de générer une interférence avec les cornes 13a et 13b du détrompeur 13. De manière avantageuse, ces ailettes 20a permettent de faciliter le vissage de la seringue de mélange 20 sur les connecteurs 5a ou 5b, en améliorant la préhension de celle-ci. De manière avantageuse, les ailettes sont contigües à la jupe externe 20b du filet « luer lock » mâle ainsi qu'à une zone conique terminale 20c du réservoir de la seringue. De manière préférentielle, ces ailettes 20a ont une forme externe en arc de cercle. Cela permet de positionner les doigts sur ces ailettes et d'assurer une bonne préhension de la seringue de mélange 20 tout en restant atraumatique.

La seringue de mélange 21 est également pourvue d'une telle géométrie.

Les connecteurs 5a, 5b et 5c comprennent un filetage 50, sur lequel un moyen de connexion permettant l'assemblage d'un dispositif médical de manière étanche se connecte. De manière préférentielle, ce moyen de connexion est un luer qui est le moyen de connexion standard de référence dans le domaine médical et qui se visse sur le filetage 50. Ce moyen de connexion se raccorde au réservoir de la seringue. Les cornes 13a et 13b permettent de dimensionner les espaces d'insertion 130 et 131 de manière qu'il soit impossible d'y introduire des seringues dont le réservoir a un diamètre supérieur à une valeur seuil correspondant à un volume maximal ou qu'il soit impossible de connecter une seringue du fait de l'incompatibilité entre au moins un moyen présent sur ladite seringue et la configuration mécanique définie par les cornes 13a et 13b .

De préférence, le détrompeur 13 permet d'empêcher le montage sur le connecteur 5c de seringues présentant un moyen rendant incompatible leur connexion avec la configuration mécanique définie par le détrompeur ou avec des seringues dont le volume est supérieur à 3 ml. De manière plus préférentielle, le détrompeur 13 permet d'empêcher le montage sur le connecteur 5c de seringues présentant au moins un moyen rendant incompatible leur connexion avec la configuration mécanique définie par le détrompeur. La présence d'ailettes à l'extrémité des seringues de mélange 20 et 21 rend leur connexion incompatible avec la configuration mécanique définie par le détrompeur comme cela est montré sur la figure 9. Ainsi, il n'est pas possible dans ce mode de réalisation, de connecter au connecteur femelle 5c, une seringue de mélange.

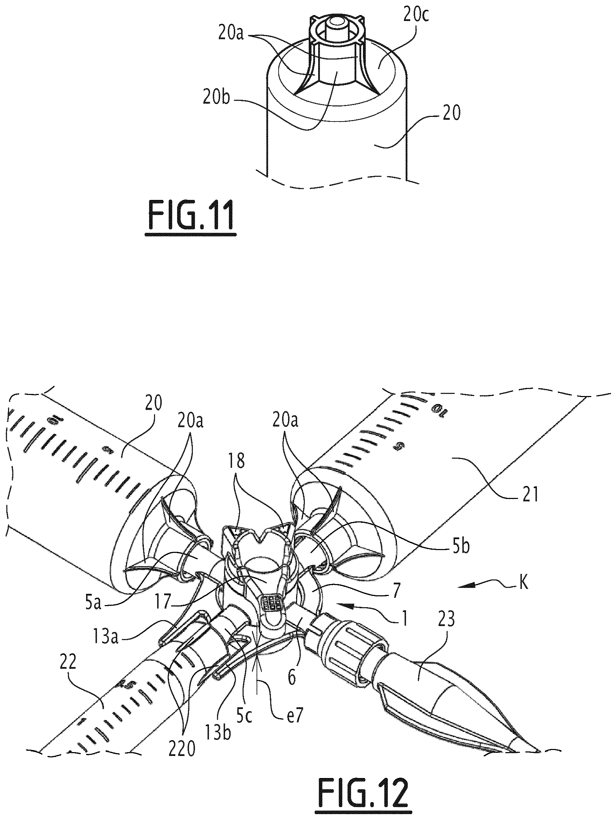

Le connecteur femelle 5c est préférentiellement adapté pour être connecté avec une seringue d'injection 22, comme cela est représenté à la figure 12. Le diamètre extérieur de cette seringue 22 et/ou l'encombrement de l'extrémité de cette seringue 22 lui permettent d'être insérée dans les espaces d'insertion 130 et 131 lors de son vissage sur le connecteur 5c. De manière préférentielle, la seringue d'injection 22 comprend à son extrémité distale des godrons de préhension 220 qui ont un encombrement inférieur à la distance D les cornes 13a et 13b.

Dans un autre mode de réalisation, une seringue de volume supérieur à 3 ml, comme une seringue de mélange 20, ne pourra pas être montée sur le connecteur 5c à cause du diamètre trop important de son réservoir.

Les connecteurs 5a et 5b ne comportent pas de détrompeur 13 et sont donc adaptés pour le montage de seringues de mélange, comme cela est représenté à la figure 12, sur laquelle deux seringues de mélange 20 et 21 sont montées sur les connecteurs 5a et 5b.

Par « seringue d'injection », on entend préférentiellement désigner une seringue de faible volume, c'est-à-dire de volume compris entre 1 ml et 3 ml. Par « seringue de mélange », on entend préférentiellement désigner une seringue de grand volume, c'est-à-dire une seringue d'un volume supérieur ou égal à 10 ml.

De façon avantageuse, les connecteurs femelles 5a et 5b sont contigus, ce qui permet la connexion de deux seringues de mélange contiguës.

De manière avantageuse, le canal de circulation de fluide 10 met en communication uniquement deux des connecteurs 5a, 5b, 5c et 6 du robinet 1. A cet effet, le canal de circulation de fluide 10 ne comprend qu'une seule voie munie de deux ouvertures 101 et 102 sur la paroi cylindrique externe 14. L'utilisation du robinet médical 1 est ainsi sécurisée. En effet, seulement deux voies sont en communication quelle que soit l'orientation du boisseau 3, ce qui permet de réaliser trois actions mais uniquement une de ces trois actions à la fois :
- le mélange d'une première substance et d'une seconde substance contenues respectivement dans les seringues de mélange 20 et 21, connectées sur les connecteurs 5a et 5b, ou
- le transfert d'une partie du mélange obtenu à partir du mélange des première et seconde substances et contenu dans l'une des seringues de mélange 20 et 21 dans la seringue d'injection 22 connectée sur le connecteur 5c, ou
- l'injection chez un patient, au travers d'un cathéter connecté sur le connecteur 6, du mélange préalablement transféré dans la seringue d'injection 22 connectée sur le connecteur 5c.

Ainsi, il ne sera pas possible d'injecter le mélange ou l'émulsion chez un patient tout en effectuant le remplissage d'une des seringues de mélange ou de la seringue d'injection 22 ou d'effectuer le mélange des première et seconde substances contenues dans l'une des seringues de mélange, préférentiellement la seringue 20, tout en transférant l'émulsion ou le mélange obtenu dans la seringue d'injection 22. De manière préférentielle, le mélange est une émulsion.

A cet effet, le canal de circulation de fluide 10 est en forme de L, ce qui permet de mettre en communication fluidique les seringues de mélange 20 et 21, ou la seringue de mélange 20 avec la seringue d'injection 22 ou la seringue d'injection 22 avec un dispositif aval tel qu'un cathéter 23 ou un micro-cathéter.

De manière avantageuse, le boisseau 3 comprend des indicateurs visuels 18 permettant de signaler à l'utilisateur du robinet 1 quels sont les connecteurs qui sont en communication par le positionnement du boisseau 3.

Dans l'exemple représenté, les indicateurs 18 sont deux flèches orientées à 90° l'une par rapport à l'autre permettant de montrer le positionnement du canal 10 de circulation de fluide.

De manière encore plus avantageuse, comme cela est représenté aux figures 6 à 8, le boisseau 3 comprend, sur le dessous d'une partie supérieure 30 qui porte le levier 17 et les indicateurs 18, une rainure circulaire 31 interrompue par des nervures 32 alignées avec les indicateurs visuels 18. Le corps 2 du robinet médical comprend sur une partie supérieure un moyen empêchant une rotation du boisseau 3 qui mettrait en communication le connecteur auquel une seringue de mélange est connectée et le connecteur auquel est connecté un dispositif aval (c'est-à-dire qui mettrait en communication les connecteurs 5b et 6). Ce moyen est formé par une nervure 202 de forme courbe et adaptée pour coulisser dans la rainure circulaire 31 entre les nervures 32.

Ce robinet permet ainsi soit d'effectuer un mélange ou une émulsion de deux substances contenues dans des seringues de mélange 20 et 21, soit de remplir une seringue d'injection 22 à partir de l'une des seringues de mélange (préférentiellement la seringue 20), soit d'injecter le mélange ou l'émulsion obtenue à un patient. Le dispositif selon l'invention est donc préférentiellement un robinet 3 voies, 4 ports.

La position des nervures 32 est préférentiellement liée à la position de la rainure circulaire 31 sur le boisseau 3. Les nervures pourraient donc, dans un autre mode de réalisation, être positionnées différemment, c'est-à-dire qu'elles pourraient ne pas être alignées avec les indicateurs visuels, à la condition que leur positionnement permette d'empêcher la mise en communication des conduits 2b et 2d.Avantageusement, le robinet médical 1 comprend au moins deux zones de préhension adaptées au positionnement d'un doigt. Ces zones de préhension permettent de saisir plus efficacement le robinet 1 et de faciliter ainsi sa manipulation lors des étapes de connexion ou de déconnexion des seringues 20, 21 et 22 ou lors de l'orientation du boisseau 3 permettant la mise en communication de tel ou tel connecteur.

Dans l'exemple représenté, le robinet 1 comprend quatre zones de préhension 15 formées par des formes circulaires planes faisant saillie du corps 2. Ces zones de préhension 15 sont réparties entre les connecteurs 5a, 5b, 5c et 6.

Le robinet médical 1 comprend une collerette de renfort 7 solidaire d'au moins deux des connecteurs du robinet médical 1. Conformément à l'invention, la collerette de renfort 7 est espacée du corps central 2 pour former une zone d'ajourage 9 autour du corps central 2. Le fait d'éloigner la collerette de renfort 7 de l'axe central du robinet 1 permet d'éloigner la zone de concentrations de contraintes et de réduire les effets des contraintes.

Lorsqu'une contrainte est appliquée à une pièce plastique, elle engendre une déformation. Sur le robinet médical 1, cette contrainte va s'appliquer par exemple entre deux connecteurs luer femelles 5b et 5c orientés à 90° l'un par rapport à l'autre, lors de la phase de mélange. La contrainte va ainsi déformer les deux connecteurs 5b et 5c par un mouvement de rapprochement. Si l'on reste dans la zone dite de « déformation élastique », le corps du robinet 1 reprend sa forme initiale dès le relâchement de cette contrainte. Si l'on dépasse la zone de déformation élastique, le matériau du robinet 1 entre alors dans une phase de déformation plastique. Au relâchement de la contrainte, le robinet 1 ne reprend pas sa forme initiale. Il conserve alors une déformation permanente. A cela va s'ajouter dans les phases de mélange, un phénomène cyclique de « déformation plastique », ce qui va provoquer une « fatigue » du matériau plastique.

Dans les zones de concentration de contraintes, les arêtes vives, les zones saillantes, les zones de perçages, les trous, des zones blanches vont commencer à se former. Elles sont la manifestation d'une zone de striction. Le matériau va perdre une partie de ses propriétés de résistance mécanique et ce phénomène va s'amplifier jusqu'à la rupture du matériau. Le matériau va alors se fissurer et le robinet se casse.

Si la contrainte appliquée est importante et si en plus s'ajoute un phénomène d'attaque chimique par une solution huileuse, le robinet 1 peut devenir directement cassant sans passer par la phase de déformation plastique. C'est le cas de la plupart des robinets standard de perfusion utilisés dans les procédures de chimio-embolisation (c-TACE).

La zone d'ajourage 9 ainsi formée évite la génération de retassures à l'intérieur du corps 2. La collerette de renfort 7 n'est donc pas en contact direct avec l'alésage 200 dans lequel le boisseau 3 est monté rotatif.

Avantageusement, la collerette de renfort 7 est solidaire de trois ou quatre connecteurs du robinet médical 1. Dans l'exemple représenté, la collerette de renfort 7 est solidaire des quatre connecteurs 5a, 5b, 5c et 6 et forme une ceinture complète autour du corps 2, améliorant ainsi la solidité du robinet 1. L'ajourage 9 qui évitera dans ce mode de réalisation tout contact de la collerette avec le fût central du robinet, permet d'éviter au maximum l'apparition de retassures.

Des tests de résistance du robinet médical 1 effectués à titre d'exemple permettent de constater que lorsque celui-ci ne comporte pas de collerette de renfort 7, la limite élastique du matériau est atteinte sous une force de cisaillement de 17 N, et que les zones de concentration de contraintes se trouve aux jointures entre les connecteurs et le corps central. Dans un tel cas, les risques de casse du robinet 1 sont importants.

En revanche, lorsque le robinet médical 1 est pourvu de la collerette 7, la limite élastique du matériau est atteinte sous une force de cisaillement de 35 N, et les zones de concentration de contraintes se trouvent aux intersections de la collerette 7 avec les connecteurs. Les risques de casse et d'apparition de retassures sont donc réduits.

Selon un mode de réalisation non représenté de l'invention, le robinet médical 1 peut comprendre seulement deux connecteurs femelles et un connecteur mâle.

Il est important que l'intersection entre la collerette de renfort 7 et chaque connecteur soit éloignée du filetage 50 du connecteur, ou plus généralement de la zone fonctionnelle du connecteur, qui est le plus souvent l'extrémité distale de ce connecteur, puisque la zone fonctionnelle est le port de connexion sur lequel ou dans lequel s'insère par exemple une seringue ou la bague de verrouillage d'une tubulure ou d'un cathéter.

La collerette de renfort 7 est réalisée dans le même matériau que le corps 2. Dans ce cas, le robinet médical 1 est formé en une pièce et dans la même matière.

En variante, la collerette de renfort 7 est réalisée dans un matériau différent du corps 2 et du boisseau 3. Dans un tel cas, le robinet médical 1 est formé en une pièce, mais implique une opération de surmoulage de la collerette 7 ce qui présente l'avantage de renforcer la tenue mécanique de cette dernière. Le surmoulage de la collerette de renfort 7 est réalisé en des matériaux plastiques « chargés ». On entend par « matériau plastique chargé », un matériau dans lequel une substance solide, non miscible, appelée « charge » a été dispersée au moment de l'injection. Préférentiellement, la charge est choisie parmi les composés de la liste suivante : les charges minérales sous forme de poudre telles que de la silice synthétique, les charges organiques telles que de la farine de bois ou d'écorce de fruits ou de la pâte de cellulose, les charges renforçantes fibreuses telles que les fibres de verre et les charges renforçantes non fibreuses telles que des microsphères de verre creuses ou de la silice synthétique. Les charges renforçantes fibreuses permettent d'améliorer les caractéristiques mécaniques, la tenue thermique et la stabilité dimensionnelle du matériau. Préférentiellement, la collerette de renfort 7 est surmoulée sur le corps 2 avec une matière chargée en fibres telles que des fibres de verre.

La collerette de renfort 7 a une largeur L7 supérieure à son épaisseur e7, ce qui permet ainsi d'améliorer le moment d'inertie et donc la tenue mécanique du robinet 1. La collerette de renfort 7 présente de préférence une largeur L7 3 à 10 fois, préférentiellement 3 à 7 fois, plus préférentiellement 3 à 5 fois plus importante que son épaisseur e7.

Au sens de la présente invention, on entend par « zone d'ajourage », une zone de vide de matière en forme de couronne entre le fût du corps 2 du robinet 1 et la collerette de renfort 7. De manière préférentielle, comme cela est représenté à la figure 2, ladite couronne dispose d'un rayon interne *rₐ*, égal au rayon externe *R_{f}* du fût du corps 2 et un rayon externe *Rₐ* égal au rayon interne *r_{c}* de la collerette de renfort 7. La différence entre *Rₐ* et *rₐ* donne la largeur *lₐ* de la zone d'ajourage 9. La largeur *lₐ* de la zone d'ajourage 9 est de préférence de 0,1 à 4 mm, plus préférentiellement de 1 à 2,5 mm et encore plus préférentiellement de 1,5 à 2,5 mm.

De manière préférentielle, la collerette de renfort 7 est une couronne de matière plastique ou d'un autre matériau tel que décrit dans la suite, réalisant la liaison entre au moins deux ports luer. La forme en couronne permet de dissiper au mieux les efforts sans générer de zone de concentration de contrainte.

Le rayon interne de la couronne *r_{c}* est égal au rayon externe de l'ajourage *Rₐ*. La largeur de la couronne *L_{c}* est de préférence de 1 à 20 mm, plus préférentiellement de 2 à 10 mm et encore plus préférentiellement de 3 à 6 mm.

L'épaisseur *e_{c}* de cette couronne de matière plastique ou d'un autre matériau tel que décrit dans la suite est de préférence de 0,5 à 8 mm, plus préférentiellement de 1 à 5 mm et encore plus préférentiellement de 1,5 à 2,5 mm.

La largeur *lₐ* de la zone d'ajourage 9 entre la collerette de renfort 7 et le fût du corps 2 est de préférence égale à l'épaisseur e7 de la collerette de renfort 7.

Cette zone d'ajourage 9 est réalisée dans l'outillage d'injection plastique par un noyau moulant. Ce noyau moulant réalisé dans un alliage d'acier subit dans des phases d'injection de la matière plastique des efforts (pression) très importants. Ces pressions peuvent être de 500 à 2500 bars et plus généralement de 500 à 1500 bars. A ces efforts s'ajoute un facteur de température de la matière plastique injectée dans la cavité et entrant en contact avec le noyau moulant, de 150 à 300°C. Cette température provoque des contraintes de dilatation fortes sur ce noyau moulant. La largeur *lₐ* encore plus préférentielle de 1,5 à 2,5 mm permet de conserver une résistance mécanique optimale du noyau moulant.

De manière préférentielle, la collerette 7 ne doit avoir aucune surface en contact avec le fût du corps 2 du robinet 1.

Préférentiellement, les moyens de préhension 15 sont situés sur la collerette de renfort 7.

Dans l'exemple représenté, les cornes 13a et 13b du détrompeur 13 s'étendent à partir de la collerette de renfort 7. En variante non représentée, les cornes 13a et 13b peuvent ne pas être connectées à la collerette de renfort 7 et s'étendre, par exemple à partir du connecteur 5c ou du corps 2.

Selon un mode de réalisation de l'invention représenté sur la figure 9, le robinet médical 1 peut ne pas comprendre de moyens de préhension ni de cornes formant un détrompeur prévus sur la collerette de renfort 7.

Le corps 2, le boisseau 3 et/ou la collerette de renfort 7 qui composent le robinet médical 1 sont réalisés à partir d'un ou plusieurs matériaux qui supportent mieux les contraintes mécaniques et chimiques. Préférentiellement, le corps 2 et la collerette de renfort 7 sont dans un matériau différent du boisseau 3. Les contraintes mécaniques sont essentiellement la déformation par cisaillement et la pression exercée sur le robinet 1 lors de sa fabrication et/ou de son utilisation. De plus, le matériau de fabrication du robinet 1 doit être résistant à tout produit pharmaceutique, y compris à des produits huileux. De préférence, le matériau doit être résistant à Lipiodol®. En outre ou alternativement, le matériau du corps 2 et/ou de la collerette de renfort 7 doit être caractérisé par un module de résistance mécanique (Module d'Young) élevé. Ainsi, le robinet médical 1, préférentiellement son corps 2 et sa collerette de renfort 7, est réalisé à partir des matériaux choisis dans la liste suivante : acrylonitrile butadiène styrène (ABS), copolymère méthyméthacrylate-acrylonitrile-butadiène styrène (MABS), polyester, polycarbonates (PC), alliages de polycarbonates, polysulfones, polyuréthanes, polyéthercétonecétone (PEKK), polyétheréthercétone (PEEK), polyaryléthercétones (PAEK), polymétacrylate de méthyle (PMMA), polyétherimides, polyamides (PA), préférentiellement les PA11 et PA12, polyméthylpentène (TPX), polysulfone (PSU), copolymères de cyclooléfines (COC), polymères de cyclooléfines (COP), fluoroplastiques autres que le polytétrafluoroéthylène (PTFE), le phosphoénolpyruvate (PEP) et des combinaisons de ces matériaux (par exemple, ABS-PC). Préférentiellement, le robinet médical 1, plus préférentiellement son corps 2 et sa collerette de renfort 7, est réalisé à partir des matériaux choisis dans la liste suivante : acrylonitrile butadiène styrène (ABS), copolymère méthyméthacrylate-acrylonitrile-butadiène styrène (MABS), polycarbonates (PC), polyétheréthercétone (PEEK), polymétacrylate de méthyle (PMMA), polyamides (PA), préférentiellement les PA11 et PA12, polyméthylpentène (TPX), polysulfone (PSU), copolymères de cyclooléfines (COC), polymères de cyclooléfines (COP). De manière encore plus préférentielle, le robinet médical 1, plus préférentiellement son corps 2 et sa collerette de renfort 7, est réalisé à partir des matériaux choisis dans la liste suivante : polyétheréthercétone (PEEK), polymétacrylate de méthyle (PMMA), polyamides (PA), préférentiellement les PA11 et PA12, polyméthylpentène (TPX) et polysulfone (PSU). De préférence, le robinet médical 1 comprend du polyamide ou est constitué de polyamide.

Préférentiellement, le boisseau 3 du robinet est réalisé dans un matériau plus tendre que le corps 2 et/ou que la collerette de renfort 7 de ce robinet. Ainsi le boisseau 3 du robinet est préférentiellement réalisé à partir des matériaux choisis dans la liste suivante : polyethylène (PE), polypropylène (PP), polyoxyméthylène (POM) ou polytéréphtalate de butylène (PBT). Ce matériau doit permettre au boisseau 3 de pouvoir légèrement se conformer dans le corps 2 du robinet. De plus, le fait que le corps 2 et que le boisseau 3 soient en des matériaux différents permet d'améliorer les propriétés de rotation du boisseau 3 dans le corps 2 du robinet. Le boisseau 3 est plus préférentiellement réalisé en POM.

Avantageusement, les connecteurs femelles 5a, 5b et 5c sont des ports d'entrée, alors que le connecteur mâle 6 est un port de sortie.

Dans un mode de réalisation, le connecteur mâle 6 comprend une bague de verrouillage 8 qui est fixe. Cette bague est donc solidaire du connecteur 6 et de façon plus générale du corps 2 du robinet 1.

Dans l'exemple représenté et de manière préférentielle, le connecteur mâle 6 comprend une bague de verrouillage 8 qui est montée par clipsage sur l'extrémité distale du connecteur mâle 6. La bague de verrouillage 8 est mobile et permet de renforcer la connexion du connecteur mâle 6 avec un connecteur femelle d'un dispositif aval (par exemple un cathéter ou un micro-cathéter). De façon plus préférentielle, la bague de verrouillage 8 tourne sur l'axe du connecteur mâle 6, qui, lui, reste fixe. Cela a comme avantage de rendre la manipulation de connexion plus aisée tout en diminuant le risque de déconnexion.

Comme cela est représenté à la figure 12, le robinet médical 1 permet de former un kit de préparation K d'un produit à injecter, préférentiellement un mélange ou une émulsion, avec deux seringues de mélange 20 et 21 et une seringue d'injection 22. Les connecteurs femelles 5a et 5b sont respectivement connectés à une première seringue de mélange 20 et à une seconde seringue de mélange 21, et le connecteur femelle 5c comprenant le détrompeur 13 est connecté à une seringue d'injection 22.

Le kit de préparation peut également comprendre un dispositif aval, tel qu'un cathéter 23 ou un micro-cathéter permettant l'injection chez un patient du produit à injecter contenu dans la seringue d'injection 22. Le cathéter 23 est monté et verrouillé sur le connecteur mâle 6 grâce à la bague de verrouillage 8.

De manière préférentielle, c'est le connecteur qui est sur la voie en communication avec un dispositif aval tel qu'un cathéter 23 ou un micro-cathéter, qui comprend un détrompeur 13. Préférentiellement, le détrompeur, selon la présente invention, empêche la connexion d'au moins un des éléments du kit, encore plus préférentiellement d'au moins deux des éléments du kit. Encore plus préférentiellement, le détrompeur 13, selon la présente invention, empêche la connexion des seringues de mélange 20, 21.

De manière préférentielle, les éléments du kit dont la connexion est empêchée par le détrompeur, sont pourvus de moyens rendant incompatible leur connexion avec le connecteur comprenant le détrompeur. Ces moyens rendant incompatible la connexion d'éléments du kit avec le connecteur comprenant le détrompeur sont de manière préférentielle, des ailettes situées à l'extrémité desdits éléments du kit.

Le kit de préparation peut également comprendre des accessoires de prélèvement des solutions facilitant le remplissage des seringues de mélange 20 et 21.

Le kit de préparation K permet de mettre en œuvre une méthode de préparation d'un mélange ou d'une émulsion destiné à l'injection chez un patient. Cette méthode comprend les étapes suivantes. Dans une première étape, deux seringues de mélange 20 et 21 sont montées sur deux connecteurs femelles correspondants 5a et 5b.

Dans une seconde étape, qui peut être réalisée simultanément à la première étape, la seringue d'injection 22 est montée sur le connecteur femelle 5c comprenant le détrompeur 13.

De manière préférentielle, les seringues de mélange 20 et 21 contiennent comme contenu respectivement une solution aqueuse et une huile, préférentiellement une huile iodée. Ladite solution aqueuse comprend au moins un agent anti-cancéreux et optionnellement au moins un agent densifiant.

De façon avantageuse, l'agent anticancéreux que peut comprendre la solution aqueuse présente dans l'une des deux seringues de mélange, est choisi parmi les anthracyclines et plus préférentiellement parmi la doxorubicine, l'épirubicine, la némorubicine et l'idarubicine. Dans un mode de réalisation avantageux, la solution aqueuse peut ainsi comprendre en outre un agent densifiant, préférentiellement au moins un produit de contraste iodé non-ionique. Le produit iodé non ionique, utilisable en tant qu'agent densifiant, est, de manière préférée, choisi parmi l'iobitridol (Xenetix®), l'iopamidol (Iopamiron®, Isovue®), l'iomeprol (Iomeron®), l'ioversol (Optiray®, Optiject®), l'iohexol (Omnipaque®), l'iopentol (Imagopaque®), l'ioxitol (Oxilan®), l'iopromide (Ultravist®), le metrizamide (Amipaque®), l'iosarcol (Melitrast®), l'iotrolan (Isovist®), l'iodixanol (Visipaque®), l'iosiménol et l'iosimide (Univist®) et un mélange de ceux-ci. L'iobitridol est le produit iodé non ionique préférentiel.

De façon avantageuse, l'huile iodée que peut contenir l'une des seringues de mélange comprend ou est constituée de dérivés d'acides gras iodés, préférentiellement d'esters éthyliques d'acides gras iodés, plus préférentiellement d'esters éthyliques d'acides gras iodés d'huile d'œillette, d'huile d'olive, d'huile de graines de colza, d'huile d'arachide, d'huile de graines de soja ou d'huile de noix, encore plus préférentiellement d'esters éthyliques d'acides gras iodés d'huile d'œillette ou d'huile d'olive. Plus préférentiellement, cette huile iodée comprend ou est constituée d'esters éthyliques d'acides gras iodés d'huile d'œillette aussi appelée pavot noir ou *Papaver somniferum var. nigrum.*

Une troisième étape consiste à mélanger la solution aqueuse contenue dans la seringue de mélange 20 avec l'huile contenue dans la seringue de mélange 21 après avoir positionné le boisseau 3 de manière à mettre en communication les seringues de mélanges 20 et 21 par l'intermédiaire des conduits 2a et 2b et en effectuant un mouvement de va-et-vient des pistons des seringues de mélange 20 et 21 jusqu'à l'obtention d'un mélange ou d'une émulsion.

Ensuite, dans une quatrième étape, une partie ou la totalité du mélange ou de l'émulsion obtenu à l'étape de mélange est transférée dans la seringue d'injection 22, après avoir positionné le boisseau 3 de manière à mettre en communication la seringue de mélange 20, dans laquelle une partie ou la totalité du mélange ou de l'émulsion obtenu à l'étape de mélange a été transférée, avec la seringue d'injection 22 par l'intermédiaire du conduit 2a et du conduit 2c.

De manière préférentielle, la quatrième étape est réalisée dès que l'utilisateur du robinet estime visuellement que le mélange ou l'émulsion est homogène.

Le cathéter 23 est monté soit simultanément avec les seringues de mélange 20 et 21 et/ou avec la seringue d'injection 22, soit après réalisation de l'étape de mélange, soit après l'étape de transfert d'une partie du mélange ou de l'émulsion dans la seringue d'injection 22. De manière préférentielle, le cathéter 23 est monté sur le connecteur mâle 6, après réalisation de cette quatrième étape de transfert.

Le positionnement du boisseau 3 de manière à ce que la seringue d'injection 22 soit en communication avec le cathéter 23 puis l'actionnement du piston de la seringue d'injection 22 permettent ensuite l'injection directe du mélange ou de l'émulsion à un patient.

Une fois que les seringues de mélange 20 et 21, la seringue d'injection 22 et le cathéter 23 ont été connectés sur le robinet 1, une étape supplémentaire de mélange du mélange ou de l'émulsion obtenu à la troisième étape peut être réalisée, dans le cas où l'utilisateur estime visuellement que le mélange ou l'émulsion a déphasé. C'est un avantage important apporté par le robinet selon l'invention par rapport aux autres robinets de l'art antérieur qui imposent des déconnexions des seringues de mélange ou de la seringue d'injection, augmentant ainsi les risques d'entrée d'air dans le dispositif d'injection.

La mise en communication de la seringue de mélange 20 avec la seringue d'injection 22 ou de la seringue d'injection 22 avec le cathéter 23 se fait en positionnant de façon adéquate le canal de circulation de fluide 10 du boisseau 3 par un mouvement de rotation du levier 17. Le positionnement correct du boisseau 3 adapté à l'étape de préparation à effectuer est vérifié à l'aide des indicateurs fléchés 18. A titre d'exemple, sur la figure 12, le boisseau 3 est positionné de manière à mettre en communication fluidique les connecteurs 5a et 5b, comme l'indiquent les flèches 18, pour permettre le mélange des contenus des seringues de mélange 20 et 21.

Le détrompeur 13 permet d'éliminer le risque qu'une seringue de mélange 20 ou 21 ne soit connectée sur le connecteur 5c destiné à la seringue d'injection 22 et de erreurs soient commises dans la préparation du mélange qui doit être injecté au patient. Grâce au détrompeur 13, il n'y a qu'un connecteur femelle sur lequel la seringue d'injection 22 peut être montée, et les seringues de mélange 20 et 21 ne peuvent être montées que sur les connecteurs femelles restants, qui sont positionnés de manière contigüe. Cela assure la fiabilité du robinet médical 1.

Dans le cas où le robinet médical 1 ne comprend que deux connecteurs femelles et un connecteur mâle, la seringue d'injection 22 est montée sur l'un des connecteurs femelle à la place de l'une des seringues de mélange 20 et 21 une fois que le mélange ou l'émulsion a été préparé et transféré dans l'une des seringues de mélange 20 et 21.

Les caractéristiques des modes de réalisation et variantes décrits ci-dessus peuvent être combinées pour former de nouveaux modes de réalisation de l'invention.

## Revendications

1. Robinet médical (1) comprenant :
- un corps (2) muni d'au moins deux connecteurs femelles (5a, 5b) et d'un connecteur mâle (6),
- un boisseau (3) mobile monté dans le corps (2), muni d'un levier (17) de rotation et comprenant un canal de circulation de fluide (10), et
- une collerette de renfort (7) solidaire d'au moins deux des connecteurs (5a, 5b, 5c, 6),
**caractérisé en ce que** la collerette de renfort (7) est espacée du corps (2) en formant une zone d'ajourage (9) autour du corps central (2).

2. Robinet médical selon la revendication 1, **caractérisé en ce qu'**il comprend quatre connecteurs dont trois connecteurs femelles (5a, 5b, 5c), et **en ce que** la collerette de renfort (7) est solidaire de trois ou quatre connecteurs (5a, 5b, 5c, 6).

3. Robinet médical selon l'une des revendications précédentes, **caractérisé en ce que** qu'il comprend au moins deux moyens de préhension (15) adaptés au positionnement d'un doigt, et **en ce que** les moyens de préhension (15) sont situés sur la collerette de renfort (7).

4. Robinet médical selon l'une des revendications précédentes, **caractérisé en ce que** la collerette de renfort (7) est réalisée dans le même matériau que le corps (2).

5. Robinet médical selon l'une des revendications 1 à 3, **caractérisé en ce que** la collerette de renfort (7) est réalisée dans un matériau différent de celui du corps (2) et de celui du boisseau (3).

6. Robinet médical selon la revendication 5, **caractérisé en ce que** la collerette de renfort (7) est réalisée en un matériau chargé en fibres et surmoulée sur le corps (2).

7. Robinet médical selon l'une des revendications précédentes, **caractérisé en ce que** le boisseau (3) est réalisé dans un matériau plus tendre que le corps (2) et/ou que la collerette de renfort (7).

8. Robinet médical selon l'une des revendications précédentes, **caractérisé en ce que** la collerette de renfort (7) a une largeur (L7) supérieure à son épaisseur (e7), de préférence une largeur (L7) 3 à 10 fois plus importante que son épaisseur (e7).

9. Robinet médical selon l'une des revendications précédentes, **caractérisé en ce qu'**au moins un des connecteurs femelles (5c) est adapté pour recevoir une seringue d'injection (22) et comprend un détrompeur (13) permettant d'empêcher le montage sur ce connecteur (5c) d'une seringue de mélange (20, 21), et **en ce que** ce détrompeur (13) est formé de deux cornes (13a, 13b) s'étendant à partir de la collerette de renfort (7) et prévues de part et d'autre du connecteur femelle (5c) adapté pour recevoir une seringue d'injection (22) et formant par rapport à ce connecteur (5c) des espaces d'insertion (130, 131).

10. Robinet médical selon la revendication 9, **caractérisé en ce que** les cornes (13a, 13b) permettent d'empêcher le montage de seringues (20, 21) pourvues d'un moyen (20a) rendant incompatible leur connexion sur le connecteur femelle (5c) adapté pour recevoir une seringue d'injection (22).

11. Kit de préparation (K) d'un produit à injecter, **caractérisé en ce qu'**il comprend:
- un robinet médical (1) selon l'une des revendications précédentes,
- deux seringues de mélange (20, 21) adaptées pour être connectées à un premier et à un deuxième connecteur femelles (5a, 5b) du robinet (1), et
- une seringue d'injection (22) adaptée pour recueillir au moins une partie du produit obtenu par le mélange des contenus des seringues de mélange (20, 21) réalisé par un mouvement de va-et-vient des pistons desdites seringues de mélange (20, 21) et adaptée pour être connectée à l'un des connecteurs femelles (5c) du robinet (1).

12. Kit de préparation selon la revendication 11, **caractérisé en ce que** l'un des connecteurs femelles (5c) du robinet (1) comprend un détrompeur (13) et **en ce que** les seringues de mélange (20, 21) comprennent un moyen (20a) rendant incompatible leur connexion avec ledit connecteur femelle (5c).

13. Kit de préparation selon la revendication 12, **caractérisé en ce que** les seringues de mélange (20, 21) comprennent des ailettes (20a) à leur extrémité.

14. Méthode de préparation d'un mélange ou d'une émulsion destiné à l'injection chez un patient, **caractérisée en ce qu'**elle comprend des étapes consistant à :
a) connecter deux seringues de mélange (20, 21) à deux connecteurs femelles correspondants (5a, 5b) d'un robinet médical (1) selon l'une des revendications 1 à 10 ;
b) mélanger une solution aqueuse contenue dans l'une des seringues de mélange (20) avec une huile contenue dans l'autre seringue de mélange (21) après avoir positionné un boisseau (3) du robinet médical (1) de manière à mettre en communication fluidique les seringues de mélanges (20, 21) l'une avec l'autre et en effectuant un mouvement de va-et-vient des pistons de ces seringues de mélange (20, 21) jusqu'à l'obtention d'un mélange ou d'une émulsion ;
c) connecter une seringue d'injection (22) à un connecteur spécifique (5c) du robinet médical (1) ou à la place des seringues de mélange (20, 21);
d) transférer une partie ou la totalité du mélange ou de l'émulsion obtenu à l'étape b) dans la seringue d'injection (22), après avoir positionné le boisseau (3) de manière à mettre en communication l'une des seringues de mélange (20, 21) avec la seringue d'injection (22).

15. Méthode de préparation selon la revendication 14, **caractérisée en ce qu'**elle comprend en outre une étape supplémentaire consistant à connecter un dispositif aval (23), de préférence un cathéter ou un micro-cathéter, à un connecteur mâle (6) du robinet médical (1), cette étape étant soit concomitante à l'étape a), soit postérieure à l'étape b), soit concomitante à l'étape c), soit postérieure à l'étape d).

## Patentansprüche

1. Medizinischer Absperrhahn (1), umfassend:
- einen Körper (2), der mit mindestens zwei Buchsenverbindern (5a, 5b) und einem Steckverbinder (6) versehen ist,
- einen in dem Körper (2) montierten beweglichen Hahnkegel (3), der mit einem Drehhebel (17) versehen ist und einen Fluidumlaufkanal (10) umfasst, und
- einen Verstärkungsbund (7), der fest mit mindestens zwei der Verbinder (5a, 5b, 5c, 6) verbunden ist,
**dadurch gekennzeichnet, dass** der Verstärkungsbund (7) unter Bildung eines durchbrochenen Bereichs (9) um den mittleren Körper (2) von dem Körper (2) beabstandet ist.

2. Medizinischer Absperrhahn nach Anspruch 1, **dadurch gekennzeichnet, dass** er vier Verbinder umfasst, drei davon Buchsenverbinder (5a, 5b, 5c), und dass der Verstärkungsbund (7) fest mit drei oder vier Verbindern (5a, 5b, 5c, 6) verbunden ist.

3. Medizinischer Absperrhahn nach einem der vorhergehenden Ansprüche, **dadurch gekennzeichnet, dass** er mindestens zwei Greifmittel (15) umfasst, die zur Positionierung eines Fingers ausgeführt sind, und dass die Greifmittel (15) an dem Verstärkungsbund (7) angeordnet sind.

4. Medizinischer Absperrhahn nach einem der vorhergehenden Ansprüche, **dadurch gekennzeichnet, dass** der Verstärkungsbund (7) in demselben Material wie der Körper (2) ausgeführt ist.

5. Medizinischer Absperrhahn nach einem der Ansprüche 1 bis 3, **dadurch gekennzeichnet, dass** der Verstärkungsbund (7) in einem Material ausgeführt ist, das sich von dem des Körpers (2) und dem des Hahnkegels (3) unterscheidet.

6. Medizinischer Absperrhahn nach Anspruch 5, **dadurch gekennzeichnet, dass** der Verstärkungsbund (7) in einem faserverstärkten Material ausgeführt ist, das an den Körper (2) angeformt ist.

7. Medizinischer Absperrhahn nach einem der vorhergehenden Ansprüche, **dadurch gekennzeichnet, dass** der Hahnkegel (3) aus einem Material ausgeführt ist, das weicher als der Körper (2) und/oder der Verstärkungsbund (7) ist.

8. Medizinischer Absperrhahn nach einem der vorhergehenden Ansprüche, **dadurch gekennzeichnet, dass** der Verstärkungsbund (7) eine Breite (L7) hat, die größer als seine Dicke (e7) ist, vorzugsweise eine Breite (L7), die um ein 3- bis 10-Faches größer als seine Dicke (e7) ist.

9. Medizinischer Absperrhahn nach einem der vorhergehenden Ansprüche, **dadurch gekennzeichnet, dass** mindestens einer der Buchsenverbinder (5c) zur Aufnahme einer Injektionsspritze (22) ausgeführt ist und eine Unverwechselbarkeitseinrichtung (13) umfasst, die es gestattet, das Montieren einer Mischspritze (20, 21) an diesen Verbinder (5c) zu verhindern, und dass diese Unverwechselbarkeitseinrichtung (13) aus zwei Vorsprüngen (13a, 13b) gebildet ist, die sich von dem Verstärkungsbund (7) erstrecken und beiderseits des Buchsenverbinders (5c) vorgesehen sind, der zur Aufnahme einer Injektionsspritze (22) ausgeführt ist, und bezüglich dieses Verbinders (5c) Einführungsräume (130, 131) bilden.

10. Medizinischer Absperrhahn nach Anspruch 9, **dadurch gekennzeichnet, dass** die Vorsprünge (13a, 13b) das Verhindern der Montage von Spritzen (20, 21) gestatten, die mit einem Mittel (20a) versehen sind, das ihre Verbindung mit dem für die Aufnahme einer Injektionsspritze (22) ausgeführten Buchsenverbinder (5c) inkompatibel macht.

11. Kit (K) zur Herstellung eines zu injizierenden Produkts, **dadurch gekennzeichnet, dass** es Folgendes umfasst:
- einen medizinischen Absperrhahn (1) nach einem der vorhergehenden Ansprüche,
- zwei Mischspritzen (20, 21), die dazu ausgeführt sind, mit einem ersten und mit einem zweiten Buchsenverbinder (5a, 5b) des Absperrhahns (1) verbunden zu werden, und
- eine Injektionsspritze (22), die dazu ausgeführt ist, mindestens einen Teil des durch das Mischen der Inhalte der Mischspritzen (20, 21) erhaltenen Produkts zu sammeln, wobei das Mischen durch eine Hin- und Herbewegung der Kolben der Mischspritzen (20, 21) erfolgt, und dazu ausgeführt ist, mit einem der Buchsenverbinder (5c) des Absperrhahns (1) verbunden zu werden.

12. Herstellungs-Kit nach Anspruch 11, **dadurch gekennzeichnet, dass** einer der Buchsenverbinder (5c) des Absperrhahns (1) eine Unverwechselbarkeitseinrichtung (13) umfasst und dass die Mischspritzen (20, 21) ein Mittel (20a) umfassen, das ihre Verbindung mit dem Buchsenverbinder (5c) inkompatibel macht.

13. Herstellungs-Kit nach Anspruch 12, **dadurch gekennzeichnet, dass** die Mischspritzen (20, 21) Rippen (20a) an ihrem Ende umfassen.

14. Verfahren zur Herstellung eines Gemischs oder einer Emulsion, das bzw. die für die Injektion in einen Patienten bestimmt ist, **dadurch gekennzeichnet, dass** es Schritte umfasst, die aus Folgendem bestehen:
a) Verbinden von zwei Mischspritzen (20, 21) mit zwei entsprechenden Buchsenverbindern (5a, 5b) eines medizinischen Absperrhahns (1) nach einem der Ansprüche 1 bis 10,
b) Mischen einer in einer der Mischspritzen (20) enthaltenen wässrigen Lösung mit einem in der anderen Mischspritze (21) enthaltenen Öl, nachdem ein Hahnkegel (3) des medizinischen Absperrhahns (1) so positioniert worden ist, dass die Mischspritzen (20, 21) miteinander in Fluidverbindung gebracht worden sind, und unter Durchführung einer Hin- und Herbewegung der Kolben dieser Mischspritzen (20, 21), bis ein Gemisch oder eine Emulsion erhalten worden ist,
c) Verbinden einer Injektionsspritze (22) mit einem spezifischen Verbinder (5c) des medizinischen Absperrhahns (1) oder anstelle der Mischspritzen (20, 21),
d) Transferieren eines Teils oder der Gesamtheit des in Schritt b) erhaltenen Gemischs oder der in Schritt b) erhaltenen Emulsion in die Injektionsspritze (22), nachdem der Hahnkegel (3) so positioniert worden ist, dass eine der Mischspritzen (20, 21) mit der Injektionsspritze (22) in Fluidverbindung gebracht wird.

15. Herstellungsverfahren nach Anspruch 14, **dadurch gekennzeichnet, dass** es ferner einen zusätzlichen Schritt umfasst, der darin besteht, eine stromabwärtige Vorrichtung (23), vorzugsweise einen Katheter oder einen Mikro-Katheter, mit einem Steckverbinder (6) des medizinischen Absperrhahns (1) zu verbinden, wobei dieser Schritt entweder gleichzeitig mit dem Schritt a) oder nach Schritt b) oder gleichzeitig mit Schritt c) oder nach Schritt d) erfolgt.

## Claims

1. Medical stopcock (1) comprising:
- a body (2) provided with at least two female connectors (5a, 5b) and a male connector (6),
- a mobile plug (3) which is mounted in the body (2), is provided with a rotation lever (17) and comprises a fluid circulation channel (10), and
- a reinforcement collar (7) rigidly connected to at least two of the connectors (5a, 5b, 5c, 6),
**characterized in that** the reinforcement collar (7) is spaced apart from the body (2), thus forming an openworked zone (9) around the central body (2).

2. Medical stopcock according to Claim 1, **characterized in that** it comprises four connectors, of which three are female connectors (5a, 5b, 5c), and **in that** the reinforcement collar (7) is rigidly connected to three or four connectors (5a, 5b, 5c, 6).

3. Medical stopcock according to either of the preceding claims, **characterized in that** it comprises at least two gripping means (15) designed for the placement of a finger, and **in that** the gripping means (15) are situated on the reinforcement collar (7).

4. Medical stopcock according to one of the preceding claims, **characterized in that** the reinforcement collar (7) is made of the same material as the body (2).

5. Medical stopcock according to one of Claims 1 to 3, **characterized in that** the reinforcement collar (7) is made of a material different than that of the body (2) and that of the plug (3) .

6. Medical stopcock according to Claim 5, **characterized in that** the reinforcement collar (7) is made of a fiber-reinforced material and is overmolded on the body (2).

7. Medical stopcock according to one of the preceding claims, **characterized in that** the plug (3) is made of a material that is softer than the body (2) and/or than the reinforcement collar (7).

8. Medical stopcock according to one of the preceding claims, **characterized in that** the reinforcement collar (7) has a width (L7) greater than its thickness (e7), preferably a width (L7) 3 to 10 times greater than its thickness (e7).

9. Medical stopcock according to one of the preceding claims, **characterized in that** at least one of the female connectors (5c) is designed to receive an injection syringe (22) and comprises a foolproofing device (13) in order to prevent a mixing syringe (20, 21) from being mounted on this connector (5c), and **in that** this foolproofing device (13) is formed by two projections (13a, 13b) which extend from the reinforcement collar (7) and are provided on each side of the female connector (5c) designed to receive an injection syringe (22) and form insertion spaces (130, 131) with respect to this connector (5c).

10. Medical stopcock according to Claim 9, **characterized in that** the projections (13a, 13b) make it possible to prevent the mounting of syringes (20, 21) that are provided with a means (20a) prohibiting their connection to the female connector (5c) designed to receive an injection syringe (22).

11. Preparation kit (K) for preparing a product to be injected, **characterized in that** it comprises:
- a medical stopcock (1) according to one of the preceding claims,
- two mixing syringes (20, 21) designed to be connected to a first and a second female connector (5a, 5b) of the stopcock (1), and
- an injection syringe (22) designed to collect at least some of the product obtained by the mixing of the contents of the mixing syringes (20, 21), effected by a reciprocating motion of the pistons of said mixing syringes (20, 21), and designed to be connected to one of the female connectors (5c) of the stopcock (1).

12. Preparation kit according to Claim 11, **characterized in that** one of the female connectors (5c) of the stopcock (1) comprises a foolproofing device (13), and **in that** the mixing syringes (20, 21) comprise a means (20a) prohibiting their connection to said female connector (5c).

13. Preparation kit according to Claim 12, **characterized in that** the mixing syringes (20, 21) comprise fins (20a) at their end.

14. Method for preparing a mixture or an emulsion intended for injection into a patient, **characterized in that** it comprises steps of:
a) connecting two mixing syringes (20, 21) to two corresponding female connectors (5a, 5b) of a medical stopcock (1) according to one of Claims 1 to 10;
b) mixing an aqueous solution, contained in one of the mixing syringes (20), with an oil contained in the other mixing syringe (21) after positioning a plug (3) of the medical stopcock (1) in such a way as to bring the mixing syringes (20, 21) into fluidic communication with each other, and effecting a reciprocating motion of the pistons of these mixing syringes (20, 21) until a mixture or an emulsion is obtained;
c) connecting an injection syringe (22) to a specific connector (5c) of the medical stopcock (1) or in place of the mixing syringes (20, 21);
d) transferring some or all of the mixture or emulsion obtained in step b) into the injection syringe (22) after positioning the plug (3) in such a way as to bring one of the mixing syringes (20, 21) into communication with the injection syringe (22).

15. Preparation method according to Claim 14, **characterized in that** it additionally comprises a supplementary step consisting in connecting a downstream device (23), preferably a catheter or a microcatheter, to a male connector (6) of the medical stopcock (1), this step being either concomitant with step a), or subsequent to step b), or concomitant with step c), or subsequent to step d).
